# EUROPEAN PATENT SPECIFICATION

(11) **EP 1 898 233 B1**
(45) Date of publication and mention of the grant of the patent: **17.08.2011**
(21) Application number: 07016327.4
(22) Date of filing: 21.08.2007
(51) Int. Cl.: G01S 15/89

(54) **Ultrasound system and method for controlling scan lines**
Ultraschallsystem und Verfahren zur Steuerung von Abtastlinien
Système à ultra-sons et procédé de contrôle de ligne de balayage

(30) Priority: 08.09.2006 KR 20060086885
(43) Date of publication of application: 12.03.2008
(73) Proprietor: MEDISON CO., LTD., Kangwon-do 250-870 (KR)
(72) Inventor: Ahn, Chi Young, Gangnam-gu Seoul 135-280 (KR); Lee, Jae Keun, Gangnam-gu Seoul 135-280 (KR)
(74) Representative: Schmid, Wolfgang

(56) References cited:
- US-A- 5 235 986
- US-A- 5 261 408
- US-A1- 2003 018 264

## Description

### BACKGROUND

### 1. Field

The present invention generally relates to an ultrasound system, and more particularly to an ultrasound system and method for controlling scan lines.

### 2. Background

An ultrasound system has become an important and popular diagnostic tool since it has a wide range of applications. Specifically, due to its non-invasive and nondestructive nature, the ultrasound system has been extensively used in the medical profession. Modem high-performance ultrasound systems and techniques are commonly used to produce two or three-dimensional diagnostic images of internal features of an object (e.g., human organs).

Conventionally, probes of an ultrasound system include a transducer elements to transmit and receive ultrasound signals having a wide bandwidth. When transducer elements are electrically excited, ultrasounds signals are generated and transmitted to an object. Ultrasound echo signals that are reflected from the object and delivered to the transducer elements are converted into electrical signals. The converted electrical signals are amplified and processed to generate ultrasound image data.

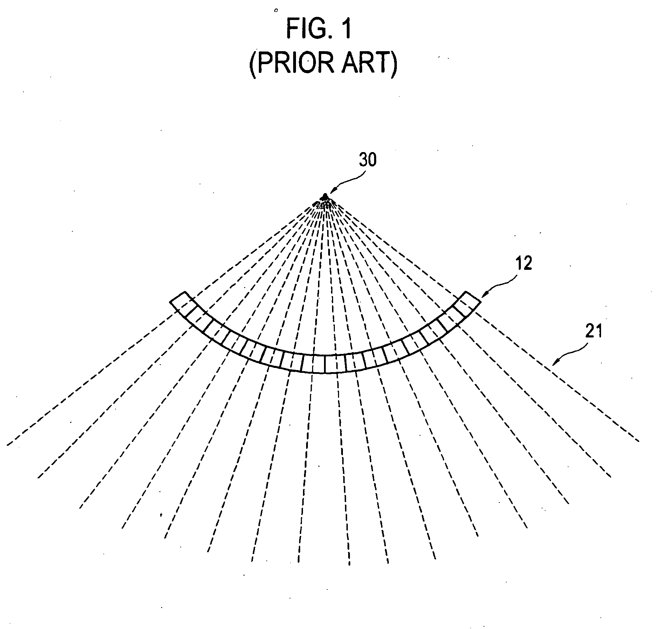

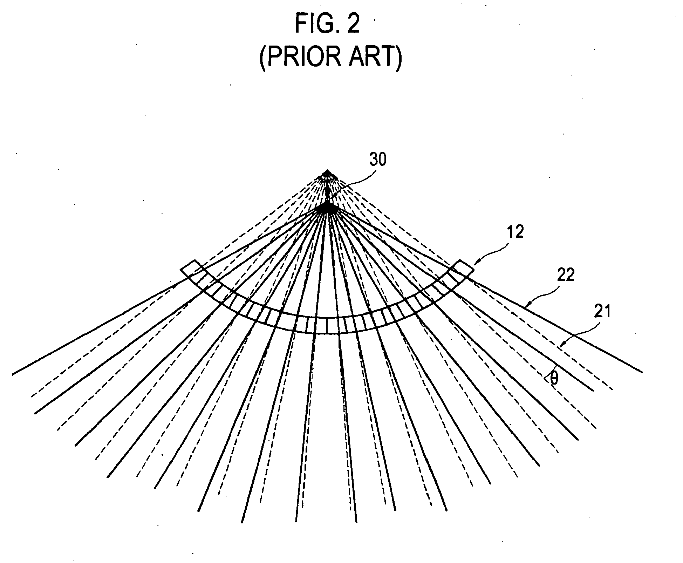

Specifically, an ultrasound system uses a curved linear probe to transmit and receive ultrasound signals. The curved linear probe transmits ultrasound signals in a radial shape such that an ultrasound image of an area, which is wider than the length of the probe, can be obtained. Fig. 1 is a geometrical diagram of scan lines for the transducer elements in a curved linear probe. As shown in Fig. 1, if each of the scan lines 21 is extended to the back of the transducer array 12, then a point ("common point") 30 at which all scan lines intersect is formed in accordance with the curvature of the surface of the transducer array 12. If the common point 30 is moved to the probe as indicated by an arrow shown in Fig. 2, then a steering angle at which each scan line 21 is steered is determined and new scan lines 22 are formed with respect to the determined steering angles to obtain an ultrasound image having a wide view angle.

However, in conventional ultrasound systems, as the location of the common point is changed, intervals between scan lines become wider and qualities of ultrasound images are deteriorated. The conventional systems also have a problem in that the image quality of an object located in the center of an ultrasound image is deteriorated when the location of the common point is changed.

US 5,261,408 and US 5,235,986 disclose acoustic scanning methods implemented by transmitting ultrasonic pressure waves and receiving return echoes on a set of spatially non-overlapping acoustic lines scanned along a transducer array with the active acoustic lines shifted and steered so that each acoustic line originates at an arbitrary point on and at an arbitrary angle to the face of the array.

### BRIEF DESCRIPTION OF THE DRAWINGS

Arrangements and embodiments may be described in detail with reference to the following drawings in which like reference numerals refer to like elements and wherein:

FIG. 1 is a geometrical diagram of scan lines for a transducer in a curved linear probe;

FIG. 2 is a geometrical diagram of scan lines for a transducer in a curved linear probe whose steering angles are adjusted;

FIG. 3 is a block diagram illustrating a structure of an ultrasound system constructed in accordance with one embodiment of the present invention;

Fig. 4 is an exemplary diagram for illustrating setting steering angles of scan lines in accordance with one embodiment of the present invention;

Fig. 5 is an exemplary diagram for illustrating setting steering angles of scan lines in accordance with another embodiment of the present invention; and

Fig. 6 is an exemplary diagram for illustrating setting steering angles of scan lines in accordance with yet another embodiment of the present invention.

### DETAILED DESCRIPTION OF THE PRESENT INVENTION

In accordance with a preferred embodiment of the present invention, there is provided an ultrasound system comprising a probe, a scan line setting unit and a control unit. The probe includes a plurality of transducer elements, which convert electrical signals into ultrasound signals for transmission along scan lines to an object and further convert the received ultrasound signals reflected from the object into electrical signals. The scan line setting unit sets a common point at which the scan lines for the transducer elements intersect. The scan line setting unit further sets a plurality of virtual common points based on the common point. The scan line setting unit also sets a steering angle of each of the scan lines for the transducer elements in accordance with the plurality of virtual common points. The control unit is programmed to control the steering of each of the scan lines for the transducer elements based on the respective steering angle.

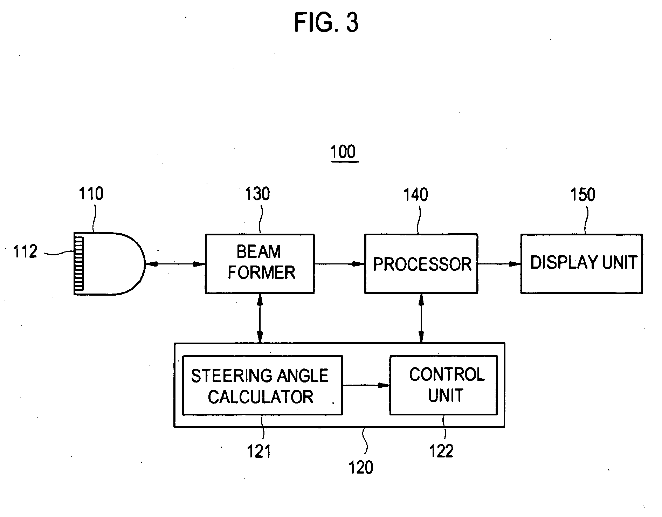

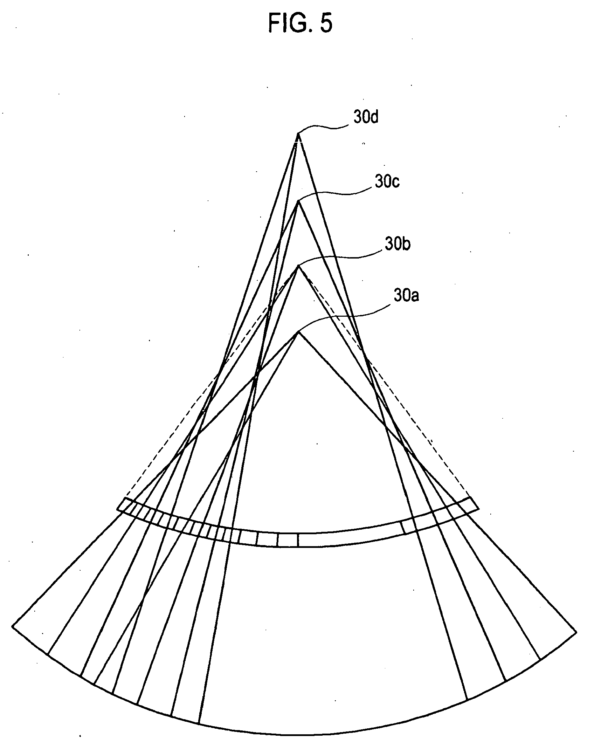

Detailed descriptions for the embodiments of the present invention are provided with reference to accompanying FIGS. 3 to 6.

As shown in FIG. 3, an ultrasound system 100 constructed in accordance with one embodiment of the present invention comprises a probe 110, a scan line setting unit 120, a beam former 130, a processor 140 and a display unit 150.

The probe 110 includes a transducer array 112 comprising a plurality of transducer elements. The probe 110 is configured to transmit ultrasound signals along the scan lines to an object and receive the ultrasound signals that are reflected from the object. In accordance with one embodiment of the present invention, the probe 110 may include a curved probe and a linear probe.

The scan line setting unit 120 includes a steering angle calculator 121 and a control unit 122, as shown in FIG. 3.

The steering angle calculator 121 is configured to set a plurality of virtual common points based on the common point at which the scan lines of the transducer elements intersect. The steering angle calculator 121 is further configured to calculate the steering angle of each of the scan lines for the transducer elements in accordance with the plurality of virtual points as set. As such, intervals between the scan lines for the transducer elements located at both ends of the transducer array 112 become wider and those for transducer elements at the center of the transducer array 112 become narrower. The steering angle calculator 121 is configured to set a plurality of virtual common points by moving the common point. The steering angle calculator 121 then divides the plurality of transducer elements into a prescribed number of groups in accordance with the number of the virtual common points as set. The steering angle calculator 121 is further configured to calculate the steering angle of each of the scan lines for the transducer elements using the virtual common point corresponding to the group to which the transducer belongs.

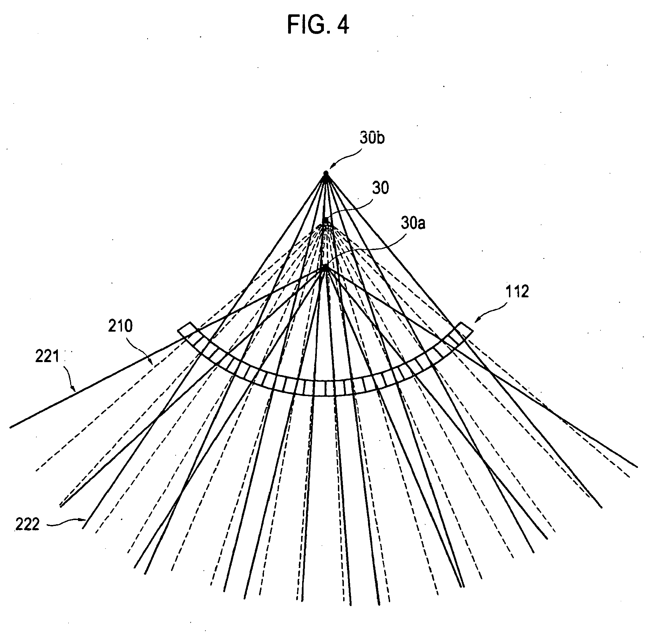

In accordance with one embodiment of the present invention, as shown in Fig. 4, the steering angle calculator 121 may be configured to set the first virtual common point 30a by moving the common point 30 toward the transducer array 112 and the second virtual common point 30b by moving the common point 30 away from the transducer array 112 based on the common point 30. The common point 30 is the point at which all scan lines 210 of the transducer elements intersect. The steering angle calculator 121 may be further configured to divide a plurality of transducer elements into two groups in accordance with the two virtual common points, which are set by the steering angle calculator 121. That is, if two virtual common points are set, then the steering angle calculator 121 may set the first transducer element group {T₁, T₃, T₅, T₇, ...} and the second transducer element group {T₂, T₄, T₆, T₈, ...} from a plurality of transducer elements {T₁, T₂, T₃, T₄, ..., Tₙ.}. The steering angle calculator 121 may also set the first virtual common point 30a corresponding to the first transducer element group and the second virtual common point 30b corresponding to the second transducer element group in order to calculate the steering angle for each of the scan line of the transducer elements. The scan lines corresponding to the first transducer element group indicated by 221 may be formed in a radial shape with the center of the virtual common point 30a. The scan lines corresponding to the second transducer element group indicated by 222 may be also formed in a radial shape with the center of the virtual common point 30b.

In accordance with another embodiment of the present invention, the steering angle calculator may be configured to set four virtual common points 30a-30d. The steering angle calculator may then set the first transducer element group {T₁, T₅, T₉, T₁₃, ...}, the second transducer element group {T₂, T₆, T₁₀, T₁₄, ...}, the third transducer element group {T₃, T₇, T₁₁, T₁₅, ...} and the fourth transducer element group {T₄, T₈, T₁₂, T₁₆, ...} from a plurality of transducer elements {T₁, T₂, T₃, T₄, ..., Tₙ.}. The steering angle calculator may be also configured to allocate four virtual common points to the transducer element groups.

While above embodiments have been explained with 2 or 4 virtual common points, it should be noted that the present invention is certainly not limited thereto. The number of virtual common points can be N. In accordance with the N virtual common points, the first transducer element group, {T₁, T_{N+1}, T_{2N+}, T_{3N+1}, ...} and the second transducer element group, {T₂, T_{N+2}, T_{2N+2}, T_{3N+2,}...} may be set from (T₁, T₂, T₃, T₄, ..., Tₙ.}. Similarly, up to the Nth transducer element group, {T_{N}, T_{2N}, T_{3N}, T_{4N}, ...}, the transducer element groups may be also set.

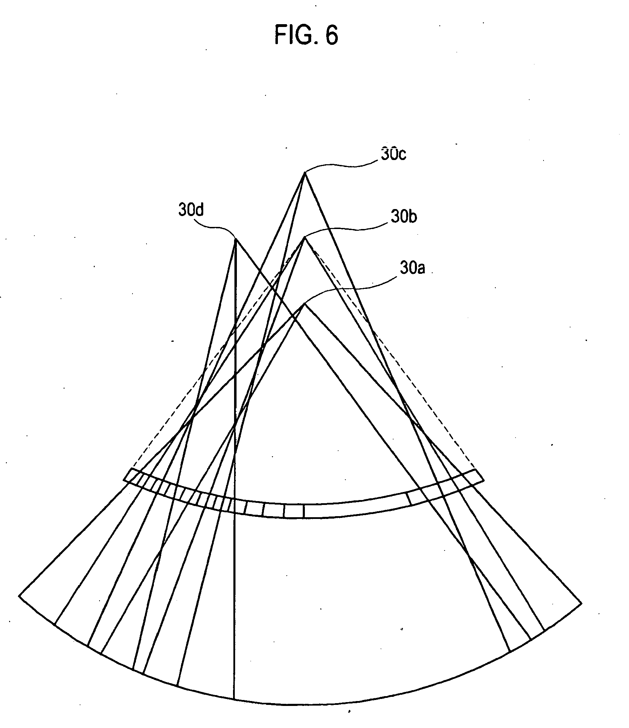

Furthermore, in the embodiments described above, a plurality of virtual common points is described to be set by moving the common point in the perpendicular direction to the transducer array 112. However, the present invention is certainly not limited to the embodiments described above. Further, a plurality of virtual common points can be set by moving the common point not only in the perpendicular direction, but also in the horizontal direction, diagonal direction, etc., as shown in Fig. 6.

The control unit 122 may be configured to control the operations of the beam former 130 and the processor 140 based on the steering angles, which are calculated from the steering angle calculator 121. The control unit 122 may be further configured to control the beam former 130 to transmit/receive the ultrasound signals along scan lines 221, 222, which are steered with the associated steering angles. Furthermore, the control unit 122 may be further configured to control the processor 140 for forming ultrasound images from signals outputted from the beam former 130 based on the information of the scan lines.

The beam former 130 may be adapted to transmit-focus ultrasound signals to the object along scan lines 221, 222 through the transducer elements 112 of the probe 110 under the control of the control unit 122. The beam former 130 may be configured to apply a time-delay to ultrasound signals and receive-focus ultrasound signals reflected from the object and received at the transducer elements.

The processor 140 may be programmed to form an ultrasound image of the object from the signals, which are outputted from the beam former 130 based on scan line information under the control of the control unit 122.

The display unit 150 may display the ultrasound image received from the processor 140.

Embodiments of the present invention may provide an ultrasound system and method for controlling scan lines. The ultrasound system may calculate the steering angle of each of the scan lines for the transducer elements based on a plurality of the virtual common points. The ultrasound system may also provide ultrasound images having a wide view angle without deteriorating the qualities of ultrasound images.

In accordance with one embodiment of the present invention, there is provided an ultrasound system according to claim 1. Furthermore, in accordance with another embodiment of the present invention, there is provided a method of controlling scan lines in an ultrasound system including a plurality of transducer elements to transmit and receive ultrasound signals along the scan lines according to claim 2.

Any reference in this specification to "one embodiment," "an embodiment," "example embodiment," etc., means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to affect such feature, structure or characteristic in connection with other ones of the embodiments.

Numerous variations and modifications are possible in the component parts and/or arrangements of the subject combination arrangement within the scope of the appended claims. In addition to variations and modifications in the component parts and/or arrangements, alternative uses will also be apparent to those skilled in the art.

## Claims

1. An ultrasound system, comprising: a probe (110) including a plurality of transducer elements (112) to transmit ultrasound signals along scan lines to an object and receive the ultrasound signals reflected from the object, the transducer elements further being configured to convert the received ultrasound signals into electrical signals, **characterized by**:
a scan line setting unit (120) configured to set a common point at which the scan lines for the transducer elements intersect and set a plurality of virtual common points by moving the set common point, the scan line setting unit (120) further being configured to divide the plurality of transducer elements into a prescribed number of groups in accordance with the number of the virtual common points and set scan lines corresponding to the respective groups to have different virtual common points, as set wherein the scan line setting unit (120) is further configured to calculate a steering angle of each of the set scan lines in accordance with the plurality of the virtual common points; and
a control unit configured to control the steering of each of the scan lines based on the respective steering angle.

2. A method of controlling scan lines in an ultrasound system including a plurality of transducer elements to transmit and receive ultrasound signals along the scan lines, the method comprising:
a) setting a plurality of virtual common points by moving a common point at which the scan lines for the transducer elements intersect;
b) dividing the plurality of transducer elements into a prescribed number of groups in accordance with the number of the virtual common points as set;
c) setting scan lines corresponding to the respective groups to have different virtual common points;
d) calculating a steering angle of each of the set scan lines; and
e) controlling the steering of each of the set scan lines based on the respective steering angle.

## Patentansprüche

1. Ultraschallsystem, welches Folgendes aufweist: eine Messsonde (110), welche eine Vielzahl von Messumformelementen (112) ausweist, um Ultraschallsignale entlang von Abtastlinien zu einem Objekt zu übertragen und die von dem Objekt reflektierten Ultraschallsignale zu empfangen, wobei die Messumformelemente des Weiteren dafür vorgesehen sind" die empfangenen Ultraschallsignale in elektrische Signale umzuwandeln, **gekennzeichnet durch** eine Abtastlinien-Einstelleinheit (120), welche dafür vorgesehen ist, einen gemeinsamen Punkt festzulegen, an welchem sich die Abtastlinien für die Messumformelemente schneiden, und um eine Vielzahl von virtuellen, gemeinsamen Punkten **durch** Bewegen des festgelegten gemeinsamen Punkts festzulegen, wobei die Abtastlinien-Einstelleinheit (120) des Weiteren dafür vorgesehen ist, die vielzahl von Mesumformelementen in eine vorbestimmte Anzahl an Gruppen in Übereinstimmung mit der Anzahl der virtuellen, gemeinsamen Punkte zu unterteilen und Abtastlinien entsprechend den jeweiligen Gruppen festzulegen, um unterschiedliche virtuelle, gemeinsame Punkte wie eingestellt zu erhalten, wobei die Abtastlinien-Einstelleinheit (120) des Weiteren dafür vorgesehen ist, einen Steuerwinkel von jeder der eingestellten Abtastlinien in Übereinstimmung mit der Vielzahl der virtuellen, gemeinsamen Punkte zu berechnen; und eine Steuereinheit, welche dafür vorgesehen ist, die Steuerung bzw. Führung von jei der der Abtastlinien basierend auf dem jeweiligen Steuerwinkel zu steuern.

2. Verfahren zur Steuerung von Abtastlinien in einem Ultraschallsystem, welches eine Vielzahl von Messumformelementen aufweist, um ultraschallsignale entlang der Abtastlinien zu übertragen und zu empfangen, wobei das Verfahren Folgendes aufweist.
a) Festlegen einer Vielzahl von virtuellen, gemeinsamen Punkten durch Bewegen eines gemeinsamen Punkts, an welchem sich die Abtastlinien für die Messumformelemente schneiden;
b) Unterteilen der Vielzahl von Messumformelementen in eine vorbestimmte Anzahl an Gruppen in Übereinstimmung mit der Anzahl der virtuellen, gemeinsamen Punkte, wie eingestellt;
c) Festlegen von Abtastlinien entsprechend den jeweiligen Gruppen, um unterschiedliche virtuelle, gemeinsame Punkte zu erhalten;
d) Berechnen eines Steuerwinkels von jeder der eingestellden Abtastlinien; und
e) Steuern der Steuerung bzw, Führung von jeder der eingestellten Abtastlinien basierend auf dem jeweiligen Steuerwinkel.

## Revendications

1. Système ultrasonore, comprenant: une sonde (110) incluant une pluralité d'éléments transducteurs (112) pour transmettre des signaux ultrasonores le long de lignes de balayage à un objet et pour recevoir les signaux ultrasonores réfléchis par l'objet, les éléments transducteurs étant en outre configurés pour convertir les signaux ultrasonores reçus en signaux électriques, **caractérisé par** :
une unité de réglage de ligne de balayage (120) configurée pour régler un point commun auquel les lignes de balayage pour les éléments transducteurs s'entrecroisent et régler une pluralité de points communs virtuels en déplaçant le point commun réglé, l'unité de réglage de ligne de balayage (120) étant en outre configurée pour diviser la pluralité d'éléments transducteurs en un nombre prescrit de groupes selon le nombre de points communs virtuels et de lignes de balayage réglées correspondant aux groupes respectifs pour avoir des points communs virtuels différents tels que réglés, dans lequel l'unité de réglage de ligne de balayage (120) est en outre configurée pour calculer un angle de direction de chacune des lignes de balayage réglées selon la pluralité de points communs, et
une unité de contrôle configurée pour contrôler la direction de chacune des lignes de balayage sur la base de l'angle de direction respectif.

2. Procédé de contrôle de lignes de balayage dans un système ultrasonore incluant une pluralité d'éléments transducteurs pour transmettre et recevoir des signaux ultrasonores le long des lignes de balayage, le procédé comprenant :
a) le réglage d'une pluralité de points communs virtuels en déplaçant un point commun auquel les lignes de balayage pour les éléments transducteurs s'entrecroisent;
b) la division de la pluralité d'éléments transducteurs en un nombre prescrit de groupes selon les points communs virtuels tels que réglés;
c) le réglage des lignes de balayage correspondant aux groupes respectifs pour avoir des points communs virtuels différents;
d) le calcul d'un angle de direction de chacune des lignes de balayage réglées; et
e) le contrôle de la direction de chacune des lignes de balayage réglées sur la base de l'angle de direction respectif.
